# EUROPÄISCHE PATENTANMELDUNG

(11) **EP 0 740 934 A1**
(43) Veröffentlichungstag der Anmeldung: **06.11.1996**
(21) Anmeldenummer: 96106143.9
(22) Anmeldetag: 19.04.1996
(51) Int. Cl.: A61K 9/14, A61K 9/20, A61K 31/44

(54) **Arzneizubereitungen mit kontrollierter Freisetzung und Verfahren zu ihrer Herstellung**

(30) Priorität: 02.05.1995 DE 19515972
(71) Anmelder: BAYER AG, 51368 Leverkusen (DE)
(72) Erfinder: Kanikanti, Venkata-Rangarao, Dr., 51381 Leverkusen (DE); Mück, Wolfgang, Dr., 42113 Wuppertal (DE); Ohm, Andreas, Dr., 41468 Neuss (DE); Kurka, Peter, Dr., 40764 Langenfeld (DE); Toppel, Gerd, Dr., 51519 Odenthal (DE)

(57) **Zusammenfassung**

Die Erfindung betrifft Arzneizubereitungen mit kontrollierter Wirkstofffreisetzung und Verfahren zu ihrer Herstellung insbesondere für schwerlösliche Wirkstoffe mit problematischer Bioverfügbarkeit.

## Beschreibung

Die Erfindung betrifft Arzneizubereitungen mit kontrollierter Wirkstofffreisetzung und Verfahren zu ihrer Herstellung insbesondere für schwerlösliche Wirkstoffe mit problematischer Bioverfügbarkeit.

Zahlreiche Arzneiwirkstoffe mit geringer Wasserlöslichkeit lassen sich nur schwierig in galenische Formulierungen überführen, die hinreichend lagerstabil sind und eine ausreichende Bioverfügbarkeit besitzen. Insbesondere Wirkstoffe mit einer Tendenz zur Rekristallisation machen Probleme, da häufig ihre Bioverfügbarkeit nicht produzierbar ist bzw. nach längeren Lagerzeiten signifikant abnimmt.

Zur Verbesserung der Sicherheit und der Compliance des Patienten ist es wünschenswert, insbesondere für Langzeitbehandlungen, die tägliche Einnahmefrequenz z.B. auf ein- bis zweimal täglich zu reduzieren. Zur Erreichung einer ausreichend hohen Bioverfügbarkeit werden schwerlösliche Wirkstoffe wie z.B. Dihydropyridine in Zubereitungen angeboten, die in kurzer Zeit hohe Freisetzungsraten zeigen, um ausreichende Plasmaspiegel zu erhalten.

Es ist bekannt, daß Wirkstoffe in amorpher bzw. gelöster Form häufig eine höher Bioverfügbarkeit besitzen als die entsprechenden kristallinen Wirkstoffe.

Die Erfindung betrifft eine oral applizierbare feste, lagerstabile Arzneizubereitung mit hoher Bioverfügbarkeit und kontrollierter Freisetzung, insbesondere lang anhaltender Wirkung enthaltend 1 Gew.-Teil des Wirkstoffs amorpher Form als Copräzipitat in 0,5 bis 10 Gew.-Teilen Polyvinylpyrrolidon, vorzugsweise 1,0 bis 5,0 Gew.-Teilen, mit einem mittleren Molekulargewicht von ca. 15.000 bis 1.000.000, vorzugsweise bis 100.000, und einer retardierenden Komponente, bestehend im wesentlichen aus 0,01 bis 15,0 Gew.-Teilen eines gelbildenden Polymers und gegebenenfalls weiteren Hilfs- und Füllstoffen.

Diese unerwartete Verbesserung der Lagerstabilität und der Bioverfügbarkeit von Wirkstoff-Copräzipitaten und diesen enthaltenden Arzneizubereitungen mit kontrollierter Freisetzung wurde als allgemeines Prinzip für schwer wasserlösliche Wirkstoffe mit geringer Bioverfügbarkeit gefunden. Beispielhaft seien genannt die Dihydropyridine wie Nifedipin, Nimodipin, Nitrendipin, Nisoldipin, Felodipin oder Nicardipin, nicht steroidale entzündungshemmende Wirkstoffe wie Ketoprofen, Ibuprofen oder Flurbiprofen oder antibakterielle Wirkstoffe, z.B. aus den Klassen Sulfonamide, Chinolone, Tetracycline oder Makrolide. Als besonders typischer Vertreter sei der Wirkstoffe Nimodipin und Nifedipin genannt. Nimodipin und Nifedipin besitzen nur eine Löslichkeit von ca. 1,0 und 4,0 mg/L. Die bisher im Markt befindlichen Nimodipin-Tabletten zeigen eine schnelle und hohe Freisetzung und erfordern eine dreimal täglich Applikation, um ausreichende Plasmaspiegel zu gewährleisten (DE 32 05 399). Die erfindungsgemäßen Retard-Tabletten zeigen ein langanhaltende Wirkung bei einer Bioverfügbarkeit, die derjenigen der schnell freisetzenden Handelsware entspricht, selbst wenn sie unter ungünstigen Bedingungen, z.B. mit einem amerikanischen Frühstück, verabreicht werden.

Bisherige Versuche eine Nimodipin-Retard-Formulierung auf Basis konventioneller Technologien, z.B. mit feinst gemahlenen Nimodipin-Kristallen herzustellen, führten nicht zum Erfolg. Bei allen Versuchen mit schwerlöslichen Wirkstoffen ergab sich eine zu geringe Bioverfügbarkeit.

Die bekannte Methode, Wirkstoffe in amorpher Form in Polymeren aufzulösen, die durch Erhitzen verflüssigt sind (Schmelzverfahren oder Schmelz-Extrusions-Verfahren), ist sehr aufwendig und benötigt einen größeren Hilfsstoffanteil (Vergleiche EP 0 240 904 und EP-A-0 240 906). Die Methode zur Amorphisierung von schwerlöslichen Wirkstoffen, nämlich das gemeinsame Aufschmelzen von Wirkstoff mit Polymeren, zeigt ebenfalls unbefriedigende Ergebnisse

Selbst bei der Verwendung von Copräzipitaten, die nach bekannten Methoden z.B. durch gemeinsame Auflösung des Wirkstoffs und des Polymers (z.B. Polyvinylpyrrolidon, PVP) hergestellt wurden (Vergleich DE 3 142 853), blieb die Bioverfügbarkeit nach Retardierung unbefriedigend. Eine andere Methode zur Erhöhung der Freisetzungsrate von schwerlöslichen Wirkstoffen ist die Adsorption der Wirkstoffe zusammen mit einem pharmakologisch unwirksamen pharmazeutischen Hilfsstoffe an ein quervernetztes Polymer. Eine exentielle Komponente all dieser Adsorbate ist das wasserunlösliche, quervernetzte Polymer. Diese Adsorbate werden mit quellbaren Polymeren gemischt zur Herstellung von Retardformulierungen (EP 0 429 187 und US 5 128 142).

Überraschenderweise wurde gefunden, daß Retard-Tabletten mit schwerlöslichen Wirkstoffen auch ohne quervernetzte Polymere hergestellt werden können. Damit wird der Hilfstoffanteil in der Tablette deutlich reduziert und es kann eine kleinere, besser schluckbare Tablette hergestellt werden.

Es zeigt sich auch, daß die so hergestellten Retard-Tabletten eine überraschend hohe Lagerstabilität und Bioverfügbarkeit zeigen. Bei der Durchführung von in-vitro-Tests zeigen sie eine fast lineare Freisetzung und in den jeweiligen Copräzipitaten bzw. Zubereitungsformen ist kein kristalliner Wirkstoff nach üblichen Methoden, z.B. Röntgenanalyse, mehr nachweisbar.

Die erfindungsgemäßen Retardformulierungen sind bioäquivalent zu den schnell freisetzenden Formulierungen, sogar wenn sie unter ungünstigen Bedingungen, z.B. zusammen mit einem amerikanischen Frühstück appliziert werden. Dies zeigt, daß die Aufnahme von Nahrung keinen negativen Einfluß auf den Effekt der erfindungsgemäßen Zubereitungen hat.

Die erfindungsgemäßen Retard-Zubereitungen können eine übliche Zusammensetzung an Wirk- und Hilfsstoffen besitzen. Vorzugsweise enthalten sie 1 Gew.-Teil Wirkstoff in amorpher Form, 0,5 bis 10 Gew.-Teile, vorzugsweise 1 bis 4 Gew.-Teile des copräzipitat-bildenden Polymers, insbesondere Polyvinylpyrrolidon (PVP) mit einem mittleren Molekulargewicht von 15.000 bis 1.000.000 und gegebenenfalls 0,01 bis 15,0 Gew.-Teilen, vorzugsweise 0,1 bis 4 Gew.-Teile eines Gelbildener, vorzugsweise Hydroxypropylmethylcellulose (HPMC). Die Weiterverarbeitung zu einer controlled release-Form erfolgt ebenfalls nach üblichen Methoden, z.B. durch die zusätzliche Verwendung von einem oder mehreren Polymeren, die in der Gegenwart von Wasser Gele bilden. Die gesamte Menge der verwendeten gelbildenden Polymere wird bestimmt durch den gewünschten Verzögerungseffekt und kann entsprechend in üblicher Weise variiert werden.

Bei dem Wirkstoff-Polymer-Copräzipitat werden bevorzugt organische Lösungsmittel eingesetzt, die den Wirkstoff und das Polymer gleichzeitig lösen. Wirkstoffe und Polymere können auch getrennt in verschiedenen Lösungsmitteln gelöst und dann die Lösungen gemischt werden. Als besonders geeignet seien niedermolekulare Halogenkohlenwasserstoffe, Ketone und/oder Alkohole mit jeweils bis zu 6 C-Atomen, insbesondere mit bis zu 4 C-Atomen genannt. Von besonderem Interesse sind Lösungsmittel, die in einfacher Weise wieder entfernt und zurückgewonnen werden können, wie Aceton, Methylenchlorid, Chloroform, Ethanol, Methanol und Isopropanol oder ihre Mischungen.

Als Copräzipitatbildner eignen sich die üblichen Polymere, insbesondere PVP mit einem mittleren Molekulargewicht von 15.000 bis 1.000.000, vorzugsweise die auf dem Markt befindlichen PVP-Arten, einschließlich ihrer Copolymeren, die unter den Bezeichnungen KOLLIDON 12PF, -17PF, -25, -30, -90 oder -VA64 im Handel sind.

Als Gelbildner eignen sich ebenfalls übliche Polymere wie vorzugsweise Celluloseether mit niedriger mittlerer oder hoher Viskosität wie Hydroxymethylcellulose, Hydroxyethylcellulose, Hydroxypropylcellulose, Hydroxybutylcellulose, Hydroxypropylmethylcellulose, Natriumcarboxymethylcellulose, Calciumcarboxymethylcellulose, Methylcellulose, Ethylcellulose und/oder Polyethylenglykole können eingesetzt werden. Celluloseether können als retardierende Komponente und auch Copräzipitatbildner eingesetzt werden.

Eine Möglichkeit zur Herstellung der Copräzipitate ist das Auflösen von einem Gewichtsteil des Wirkstoffs mit 0,5 bis 10 Gew.-Teilen, vorzugsweise 1 bis 4 Gew.-Teilen, des Copräzipitat-bildenden Polymers (PVP) und gegebenenfalls der Celluloseether in einer solchen Menge organischer Lösungsmittel, in denen sich beide Komponenten lösen oder getrennte Lösungen von Wirkstoff und Polymer in unterschiedlichen Lösungsmitteln und Vereinigen dieser Lösungen zu einer gemeinsamen. Diese Lösung kann direkt zum Granulieren der 0,01 bis 13 Gew.-Teilen, vorzugsweise 0,1 bis 2 Gew.-Teile Gelbildener und/oder weitere Hilfsstoffe verwendet werden oder alternativ können die weiteren Hilfsstoffe/Gelbildner in dieser Lösung suspendiert und anschließend das organische Lösungsmittel entfernt werden. Die Entfernung des Lösungsmittels kann nach üblichen Methoden, z.B. bei erhöhten Temperaturen zwischen 70 und 150°C, vorzugsweise zwischen 90 und 120°C unter Druck von 5 bis 200 mbar erfolgen. Auch übliche Sprühtrocknungsverfahren sind hierzu geeignet. Erforderlichenfalls kann diese Trocknung unter Stickstoffatmosphäre stattfinden. Die Dauer der Trocknung bestimmt durch Art und Menge des Lösungsmittels und den übrigen Komponenten und kann zwischen 30 Minuten und 3 Tagen liegen. Vorzugsweise erfolgt die Trocknung in 2 bis 48 Stunden. Das erhaltene trockene Copräzipitat kann gegebenenfalls noch in einem Trockenkompaktierschritt unterzogen werden um z.B. ein gewünschtes Schüttvolumen von ca. 1,5 bis 3,5 ml/g zu erzielen.

Die so erhaltene Copräzipitat-Vormischung kann entweder direkt oder durch Zugabe einer zusätzlichen retardierenden Komponente bzw. Hilfsstoffen in fertige Arzneizubereitungen überführt werden. Bei der Herstellung von Retard-Zubereitungen erfolgt dies z.B. durch Mischen von 1 Gew.-Teil Vormischung mit bis zu 10 Gew.-Teilen eines gelbildenden Polymers und gegebenenfalls weiteren Hilfsstoffen. Zusätzliche Gelbildner werden nur noch bei Bedarf eingesetzt, d.h. die Gesamt-Gelbildner-Menge kontrolliert die Freisetzungsrate. Diese Mischung wird anschließend nach üblichen Methoden zu Tabletten oder sonstigen festen Zubereitungsformen weiterverarbeitet.

Als gelbildende Polymere der retardierenden Komponente seien vorzugsweise genannt Hydroxyethylcellulose (HEC), Hydroxypropylcellulose (HPC), Hydroxypropylmethylcellulose (HPMC), Natriumcarboxymethylcellulose (Na CMC), Methylcellulose (MC) und Ethylcellulose (EC). Besonders geeignet sind Cellulose-Derivate auf Basis von HPMC. Auch andere Gelbildner wie Natriumalginat, Karrageenane, Galactomanon, Agar-Agar, Gummiarabicum, Guargummi, Xanthangummi und Carboxypolymethylen oder Derivate von Stärken, Pektinen, Chitin, Polyethylenglykol, Polyvinylalkohol, Copolymere von Acryl- und Methacrylsäureestern und deren Mischungen können eingesetzt werden Vorzugsweise werden solche Polymere verwendet, die eine Viskosität von 2 % w/w-Lösung bei 20°C von mindestens 15 mPa s⁻¹ besitzen.

Die erfindungsgemäßen Controlled-release-Zubereitungen enthalten zweckmäßigerweise 10 bis 400 mg Wirkstoff pro Einzelzubereitung. Im Falle von Retardzubereitungen beträgt die Dosierung 20 bis 400 mg, vorzugsweise 30 bis 120 mg Wirkstoff pro Einzelgabe.

Die erfindungsgemäßen Zubereitungen ergeben vor allem dann positive Effekte, wenn der Lösungsmittelrestgehalt in dem Copräzipitat nicht zu hoch ist. Vorzugsweise sollte der Lösungsmittelrestgehalt nicht größer als 1 Gew.-%, bezogen auf das Gewicht des Copräzipitats, vorzugsweise weniger als 0,8 Gew.-% betragen.

Die erfindungsgemäßen retardierten Copräzipitat-Tabletten, die zum Beispiel 90 mg amorphes Nimodipin enthalten, zeigen extrem gute Lagerstabilität. Bei Kenntnis des Standes der Technik konnte nicht erwartet werden, daß eine stabile retardierte Copräzipitat-Tablette mit zufriedenstellender Bioverfügbarkeit für schwerlösliche Wirkstoffe wie z.B. Nimodipin oder Nifedipin ohne wasserunlösliche quervernetzte Polymere herstellbar ist.

Die Retard-Tabletten können anschließend mit einem Lichtschutzlack versehen werden, damit der Wirkstoff nicht durch Lichteinwirkung abgebaut wird. Die Lackierung erfolgt z.B. durch Aufsprühen einer wäßrigen Suspension, bestehend aus: HPMC (Filmbildner), PEG (Weichmacher), Titandioxid und Eisenoxid (lichtstreuende und absorbierende Pigmente). Während der Lackierung wird heiße Luft zum Abtrocknen des Wassers auf das Tablettenbett gerichtet.

Die folgenden Ausführungsbeispiele erläutern den Erfindungsgegenstand ohne ihn zu beschränken.

### Herstellung von Wirkstoff-Copräzipitat/Zwischenprodukt

### Beispiel 1

1 kg Nimodipin wird zusammen mit 2,5 kg PVP (MG 29000) in genügend Aceton gelöst. Anschließend wird das Aceton bei erhöhter Temperatur und vermindertem Druck entfernt bis zu einem Lösungsmittelrestgehalt von weniger als 0,6 %. Das getrocknete Copräzipitat wird gesiebt und das erhaltene feine Pulver kann nach üblichen Methoden zu Tabletten weiterverarbeitet werden.

### Beispiel 2

In Analogie zu Beispiel 1 wird ein Copräzipitat mit 1 Teil Nimodipin und 2,5 Teilen PVP und 1 Teil HPMC 50 CP hergestellt. Anstelle von Aceton wird das Lösungsmittel Isopropanol eingesetzt.

### Beispiel 3

In Analogie zu Beispiel 2 wird anstelle von HPMC 50 CP das Polymer HPMC 1500 CP eingesetzt.

### Beispiel 4

In Analogie zu Beispiel 2 wird anstelle von HPMC 50 CP das Polymer HPMC 4000 CP eingesetzt.

### Beispiel 5

In Analogie zu Beispiel 1 werden 2,5 kg PVP mit dem mittleren Molekulargewicht von 45.000 eingesetzt.

### Beispiel 6

In Analogie zu Beispiel 2 werden 1,5 kg PVP mit dein Molekulargewicht von 1.000.000 eingesetzt und auch anstelle von Isopropanol das Lösungsmittel Ethanol eingesetzt. Ethanolische Lösung von Nimodipin und PVP ist klar, bevor HPMC 50 CP zugegeben wird.

### Beispiel 7

Zusammensetzung ist wie in Beispiel 4. Nimodipin und PVP werden in Aceton gelöst bzw. Nimodipin in Aceton und PVP in Ethanol separat gelöst und dann gemischt. Diese klare Lösung wird benutzt für die Granulation mit HPMC 4000 CP. Anschließend wird das Lösungsmittel nach üblichen Methoden entfernt.

### Beispiel 8

In Analogie zu Beispiel 7 anstelle von Nimodipin wird der Wirkstoff Nifedifin eingesetzt.

### Beispiel 9

In Analogie zu Beispiel 8 werden 1,5 kg PVP mit dem Molekulargewicht von 1.000.000 eingesetzt.

### Beispiel 10

In Analogie zu Beispiel 8 wird anstelle von HPMC 4.000 CP das Polymer HPMC 100.000 CP eingesetzt. Es werden 3,0 kg PVP (MG 29.000) eingesetzt.

### Beispiel 11

In Analogie zu Beispiel 8 wird anstelle von PVP das Copolymer (Copolyvidon/Kollidon VA 64) eingesetzt.

### Beispiel 12

In Analogie zu Beispiel 1 wird anstelle von Aceton das Lösungsmittel Ethanol eingesetzt und anstelle von PVP das Polymer HPMC 15000 CP eingesetzt.

### Beispiel 13

In Analogie zu Beispiel 1 wird anstelle von Aceton das Lösungsmittel Isopropanol eingesetzt.

### Beispiel 14

In Analogie zu Beispiel 1 wird anstelle von Aceton das Lösungsmittel Methylenchlorid eingesetzt.

### Beispiel 15

Das Copräzipitat, welches analog Beispiel 7 erhalten wurde, wird durch Sprühgranulation bei Temperaturen zwischen 60 bis 120°C im Wirbelsicht-Granulator unter vermindertem Druck (<300 mbar) hergestellt.

### Beispiel 16

In Analogie zu Beispiel 1 wird anstelle von Nimodipin der Wirkstoff Nifedipin und 2,0 kg PVP (MG 29.000) eingesetzt.

### Beispiel 17

In Analogie zu Beispiel 1 wird anstelle von Nimodipin der Wirkstoff Ketoprofen eingesetzt.

### Beispiel 18

In Analogie zu Beispiel 1 werden 1,5 kg PVP mit dem mittleren Molekulargewicht von 1.000.000 eingesetzt. Anstelle von Aceton wird Ethanol eingesetzt.

### Beispiel 19

In Analogie zu Beispiel 12 wird anstelle von HPMC das Polymer Hydroxypropylcellulose (HPC) eingesetzt).

### Beispiel 20

In Analogie zu Beispiel zu Beispiel 19, wird anstelle von HPC das Polymer Methylcellulose (MC) eingesetzt.

### Beispiel 21

In Analogie zu Beispiel 20, wird anstelle von MC das Polymer Hydroxyethylcellulose (HEC) eingesetzt.

### Beispiel 22

In Analogie zu Beispiel 18, anstelle von Nimodifin der Wirkstoff Nifedifin eingesetzt.

### Beispiel 23

In Analogie zu Beispiel 5, anstelle von Nimodifin der Wirkstoff Nifedifin eingesetzt.

### Beispiel 24

In Analogie zu Beispiel 12, anstelle von Nimodifin der Wirkstoff Nifedifin eingesetzt.

### Beispiel 25

In Analogie zu Beispiel 19, anstelle von Nimodifin der Wirkstoff Nifedifin eingesetzt.

### Beispiel 26

In Analogie zu Beispiel 20, anstelle von Nimodifin der Wirkstoff Nifedifin eingesetzt.

### Beispiel 27

In Analogie zu Beispiel 21, anstelle von Nimodifin der Wirkstoff Nifedifin eingesetzt.

### Einführung einer retardierenden Komponente und/oder Weiterverarbeitung als feste Arzneiform

### Beispiel 28

75,76 Gew.-Teile des Copräzipitatpulvers, erhalten gemäß Beispiel 1, werden mit 24,05 Gew.-Teilen HPMC-4000 CP vermischt und dann gemischt mit 0,19 Gew.-Teilen Magnesiumstearat und danach zu Tabletten gepreßt, die jeweils 90 mg Nimodipin enthalten. Die erhaltenen Tabletten werden anschließend lackiert.

### Beispiel 29

74,12 Gew.-Teile des Copräzipitatpulvers, erhalten gemäß Beispiel 1, werden mit 21,74 Gew.-Teilen HPMC-4.000 CP, 3,95 Gew.-Teilen HPMC-1.500 CP und 0,19 Gew.-Teilen Magnesiumstearat gemischt und danach zu Tabletten gepreßt, die jeweils 90 mg Nimodipin enthalten. Die erhaltenen Tabletten werden in Analogie zu Beispiel 28 lackiert.

### Beispiel 30

67,15 Gew.-Teile des Copräzipitatpulvers, erhalten gemäß Beispiel 18, werden mit 32,6 Gew-Teilen HPMC 1500 CP und 0,25 Gew.-Teilen Magnesiumstearat gemischt und danach zu Tabletten gepreßt die jeweils 90 mg Nimodipin erhalten. Die erhaltenen Tabletten werden in Analogie zu Beispiel 28 lackiert.

### Beispiel 31

84,2 Gew.-Teile des Copräzipitatpulvers, erhalten gemäß Beispiel 2, werden mit 15,6 Gew.-Teilen HPMC-4.000 CP vermischt, trocken kompaktiert, gesiebt und dann gemischt mit 0,2 Gew.-Teilen Magnesiumstearat und danach zu Tabletten gepreßt. Die erhaltenen Tabletten werden anschließend lackiert.

### Beispiel 32

53,8 Gew.-Teile des Copräzipitatpulvers, erhalten gemäß Beispiel 23, werden mit 41 Gew.-Teilen HPMC-4000 CP und 5 Gew.-Teilen Lactose und 0,2 Gew.-Teilen Magnesiumstearat gemischt und danach zu Tabletten gepreßt. Die erhaltenen Tabletten werden anschließend lackiert.

### Beispiel 33

49,9 Gew.-Teile des Copräzipitatpulvers, erhalten gemäß Beispiel 16, werden mit gleicher Menge von HPMC-15000 CP und 0,2 Gew.-Teilen Magnesiumstearat gemischt und danach zu Tabletten gepreßt. Die erhaltenen Tabletten werden anschließend lackiert.

### Beispiel 34

88 Gew.-Teile des Copräzipitatpulvers, erhalten gemäß Beispiel 10, werden mit 11,7 Gew.-Teilen HPMC 100.000 CP und 0,3 Gew.-Teilen Magnesiumstearat gemischt und danach zu Tabletten gepreßt. Die erhaltenen Tabletten werden anschließend lackiert.

## Patentansprüche

1. Oral applizierbare feste Arzneizubereitung mit hoher Bioverfügbarkeit, kontrollierter Freisetzung und hoher Lagerstabilität, enthaltend 1 Gew.-Teil des Wirkstoffs in amorpher Form als Copräzipitat in 0,5 bis 10 Gew.-Teilen Polyvinylpyrrolidon mit einem mittleren Molekulargewicht von ca. 15.000 bis 1.000.000 und gegebenenfalls einer retardierenden Komponente, bestehend im wesentlichen aus 0,01 bis 15,0 Gew.-Teilen eines gelbildenden Polymers und gegebenenfalls weiteren Hilfs- und Zusatzstoffen.

2. Oral applizierbare feste Arzneizubereitung gemäß Anspruch 1, dadurch gekennzeichnet, daß der Wirkstoff in amorpher Form als Copräzipitat mit 1,0 bis 4 Gew.-Teilen Polyvinylpyrrolidon mit einem mittleren Molekulargewicht von ca. 15.000 bis 100.000 vorliegt.

3. Oral applizierbare feste Arzneizubereitung gemäß Anspruch 1, dadurch gekennzeichnet, daß sie als retardierende Komponente 0,1 bis 10 Gew.-Teile gelbildende Celluloseether enthalten.

4. Oral applizierbare feste Arzneizubereitung gemäß Anspruch 1, dadurch gekennzeichnet, daß sie Wirkstoffe aus der Gruppe der Dihydropyridine enthalten.

5. Verfahren zur Herstellung von Arzneizubereitung gemäß Anspruch 1, dadurch gekennzeichnet, daß man 0,5 bis 10 Gew.-Teile Polyvinylpyrrolidon mit einem mittleren Molekulargewicht von ca. 15 000 bis 1 000 000 und 1 Gew.-Teil Wirkstoff in geeigneten organischen Lösungsmitteln löst und diese Lösung entweder direkt zum Granulieren der 0,01 bis 15 Gew.-Teilen eines gelbildenden Polymers und/oder weitere Hilfsstoffe verwendet oder die genannten Gelbildner und/oder Hilfsstoffe in dieser Lösung suspendiert und anschließend das Lösungsmittel nach üblichen Methoden entfernt und die erhaltene Copräzitat-Vormischung, gegebenenfalls unter Verwendung weiterer Hilfsmittel nach üblichen Methoden in eine geeignete feste Arzneizubereitung überführt.
